(19) Europäisches Patentamt
European Patent Office
Office européen des brevets

(11) **EP 0 951 910 A2**

(12) **EUROPEAN PATENT APPLICATION**

(43) Date of publication:
27.10.1999 Bulletin 1999/43

(51) Int Cl.[6]: **A61K 38/28**

(21) Application number: 99300111.4

(22) Date of filing: **07.01.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **08.01.1998 US 70752 P**

(71) Applicant: **ELI LILLY AND COMPANY**
**Indianapolis, Indiana 46285 (US)**

(72) Inventors:
• **Dimarchi, Richard Dennis**
**Carmel, Indiana 46033 (US)**

• **Harrison, Roger Garrick**
**Zionsville, Indiana 46077 (US)**
• **Wolff, Ronald Keith**
**Carmel, Indiana 46033 (US)**

(74) Representative: **Denholm, Anna Marie et al**
**Eli Lilly and Company Limited,**
**Lilly Research Center,**
**Erl Wood Manor**
**Windlesham, Surrey GU20 6PH (GB)**

(54) **Method for administering monomeric insulin analogs**

(57)    The claimed invention relates to a method of administering an insulin analog by inhalation, a method for treating diabetes by administering an insulin analog by inhalation, and a method for treating hyperglycemia by administering an insulin analog by inhalation.

**Description**

This invention relates generally to methods of treating humans suffering from diabetes mellitus. More specifically, this invention relates to the pulmonary delivery of monomeric insulin analogs for systemic absorption through the lungs to significantly reduce or eliminate the need for administering monomeric insulin analogs by injection.

Since the introduction of insulin in the 1920s, continuous strides have been made to improve the treatment of diabetes mellitus. Major advances have been made in insulin purity and availability and various formulations with different time-actions have also been developed. A non-injectable form of insulin is desirable for increasing patient compliance with intensive insulin therapy and lowering their risk of complications.

Diabetes mellitus is a disease affecting approximately 6% of the world's population. Furthermore, the population of most countries is aging and diabetes is particularly common in aging populations. Often, it is this population group which experiences difficulty or unwillingness to self-administer insulin by injection. In the United States approximately 5% of the population has diabetes and approximately one-third of those diabetics self-administer one or more doses of insulin per day by subcutaneous injection. This type of intensive therapy is necessary to lower the levels of blood glucose. High levels of blood glucose, which are the result of low or absent levels of endogenous insulin, alter the normal body chemistry and can lead to failure of the microvascular system in many organs. Untreated diabetics often undergo amputations and experience blindness and kidney failure. Medical treatment of the side effects of diabetes and lost productivity due to inadequate treatment of diabetes is estimated to have an annual cost of about $40 billion in the United States alone.

The nine year Diabetes Control and Complications Trial (DCCT), which involved 1,441 type 1 diabetic patients, demonstrated that maintaining blood glucose levels within close tolerances reduces the frequency and severity of diabetes complications. Conventional insulin therapy involves only two injections per day. The intensive insulin therapy in the DCCT study involved three or more injections of insulin each day. In this study, the incidence of diabetes side effects was dramatically reduced. For example, retinopathy was reduced by 50-76%, nephropathy by 35-56%, and neuropathy by 60% in patients employing intensive therapy.

Unfortunately, many diabetics are unwilling to undertake intensive therapy due to the discomfort associated with the many injections required to maintain close control of glucose levels. This type of therapy can be both psychologically and physically painful. Upon oral administration, insulin is rapidly degraded in the GI tract and is not absorbed into the blood stream. Therefore, many investigators have studied alternate routes for administering insulin, such as oral, rectal, transdermal, and nasal routes. Thus far, however, these routes of administration have not resulted in effective insulin absorption.

It has been known for a number of years that some proteins can be absorbed from the lung. In fact, administration of insulin as an inhalation aerosol to the lung was first reported by Gaensslen in 1925. Despite the fact that a number of human and animal studies have shown that some insulin formulations can be absorbed through the lungs, pulmonary delivery has not received wide acceptance as a means for effectively treating diabetes. This is due in part to the small amount of insulin which is absorbed relative to the amount delivered. In addition, investigators have observed a large degree of variability in the amount of insulin absorbed after pulmonary delivery of different insulin formulations or even doses of the same formulation delivered at different times.

Thus, there is a need to provide an efficient and reliable method to deliver insulin by pulmonary means. This need is particularly apparent for patients undergoing aggressive treatment protocols using rapid-acting human monomeric insulin analogs. Efficient pulmonary delivery of fast-acting human monomeric insulin analogs would have the effect of rapidly reducing blood glucose concentrations should the need arise, such as after a meal or after a prolonged period without insulin therapy.

It is clear that not all proteins can be efficiently absorbed in the lungs. There are numerous factors which impact whether a protein can be effectively delivered through the lungs. Absorption through the lungs is dependent to a large extent on the physical characteristics of the particular therapeutic protein to be delivered. Thus, even though pulmonary delivery of regular human insulin has been observed, the physical differences between regular human insulin and rapid-acting monomeric insulin analogs made it unclear whether these analogs could be effectively delivered through a pulmonary route.

Efficient pulmonary delivery of a protein is dependent on the ability to deliver the protein to the deep lung alveolar epithelium. Proteins that are deposited in the upper airway epithelium are not absorbed to a significant extent. This is due to the overlying mucus which is approximately 30 - 40 μm thick and acts as a barrier to absorption. In addition, proteins deposited on this epithelium are cleared by mucociliary transport up the airways and then eliminated via the gastrointestinal tract. This mechanism also contributes substantially to the low absorption of some protein particles. The extent to which proteins are not absorbed and instead eliminated by these routes depends on their solubility, their size, as well as other less understood characteristics.

It is difficult to predict whether a therapeutic protein can be rapidly transported from the lung to the blood even if the protein can be successfully delivered to the deep lung alveolar epithelium. Absorption values for some proteins delivered through the lungs have been calculated and range from fifteen minutes for parathyroid hormone (fragment 1-34) to 48 hours for glycosylated $\alpha$1-antitrypsin. Because of the broad spectrum of peptidases which exist in the lung, a longer absorption time increases the possibility that the protein will be significantly degraded or cleared by mucociliary transport before absorption.

Insulin is a peptide hormone with a molecular weight of approximately 5,800 Daltons. In the presence of zinc, human insulin self-associates into a stable hexamer form. The dissociation of the stable hexamer is believed to be the rate limiting step in the absorption of insulin from the subcutaneous injection site to the blood stream. Rapid-acting insulin analogs, however, do not readily form stable hexamers. These analogs are known as monomeric insulin analogs because they are less prone to self-associate to stable higher-ordered complexes. This lack of self-association is due to modifications in the amino acid sequence of human insulin that decrease association by disrupting the formation of dimers. Unfortunately, the modifications to insulin which cause these analogs to be monomeric, also result in non-specific aggregation of monomers. This non-specific aggregation can render the analogs insoluble and unstable.

Thus, because of the inherent instability of monomeric insulin analogs, the possibility of forming insoluble insulin analog precipitates, the physical differences between insulin and monomeric insulins analogs, and the high degree of variability in the absorption of regular human insulin delivered through the lungs, it was surprising that aerosolized monomeric insulin analog formulations could be reproducibly and effectively delivered through the lungs. Most advantageous and unexpected is the discovery that, in contrast to the data obtained with regular human insulin, a change in inhaled volume does not lead to detectable differences in either the pharmacokinetics or pharmacodynamics of the monomeric insulin analogs, particularly $Lys^{B28}Pro^{B29}$-human insulin. In addition, it was surprising that $Lys^{B28}Pro^{B29}$-human insulin is absorbed at least as rapidly from the lung, after delivery as following subcutaneous administration.

The present invention relates to a method for administering a monomeric insulin analog comprising, administering an effective amount of the monomeric insulin analog to a patient in need thereof by pulmonary means. The present invention also relates to a method for treating diabetes comprising, administering an effective dose of a monomeric insulin analog to a patient in need thereof by pulmonary means. Another aspect of the invention relates to a method for treating hyperglycemia comprising, administering an effective dose of a monomeric insulin analog to a patient in need thereof by pulmonary means. Preferably, the monomeric insulin analogs are delivered by inhalation and to the lower airway of the patient.

The monomeric insulin analogs can be delivered in a carrier, as a solution or suspension, or as a dry powder, using any of a variety of devices suitable for administration by inhalation. Preferably, the monomeric insulin analogs are delivered in a particle size effective for reaching the lower airways of the lung. A preferred monomeric insulin analog particle size is below 10 microns. An even more preferred monomeric insulin analog particle size is between 1 and 5 microns.

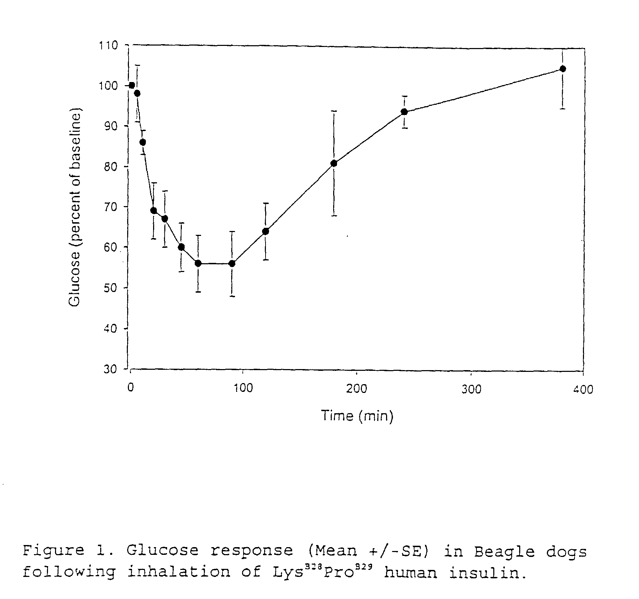

Figure 1 graphs the mean glucose response in beagle dogs versus time after aerosol delivery of $Lys^{B28}Pro^{B29}$-human insulin.

The term "insulin" as used herein refers to mammalian insulin, such as bovine, porcine or human insulin, whose sequences and structures are known in the art. The amino acid sequence and spatial structure of human insulin are well-known. Human insulin is comprised of a twenty-one amino acid A-chain and a thirty amino acid B-chain which are cross-linked by disulfide bonds. A properly cross-linked human insulin contains three disulfide bridges: one between position 7 of the A-chain and position 7 of the B-chain, a second between position 20 of the A-chain and position 19 of the B-chain, and a third between positions 6 and 11 of the A-chain.

The term "insulin analog" means proteins that have an A-chain and a B-chain that have substantially the same amino acid sequences as the A-chain and B-chain of human insulin, respectively, but differ from the A-chain and B-chain of human insulin by having one or more amino acid deletions, one or more amino acid replacements, and/or one or more amino acid additions that do not destroy the insulin activity of the insulin analog.

One type of insulin analog, "monomeric insulin analog," is well known in the art. These are fast-acting analogs of human insulin, including, for example, monomeric insulin analogs wherein:
a) the amino acyl residue at position B28 is substituted with Asp, Lys, Leu, Val, or Ala, and the amino acyl residue at position B29 is Lys or Pro; b) the amino acyl residues at positions B28, B29, and B30 are deleted; or c) the amino acyl residue at position B27 is deleted. A preferred monomeric insulin analog is $Asp^{B28}$. An even more preferred monomeric insulin analog is $Lys^{B28}Pro^{B29}$.

Monomeric insulin analogs are disclosed in Chance, et al., U.S. Patent No. 5,514,646; Chance, et al., U.S. Patent Application Serial No. 08/255,297; Brems, et al., Protein Engineering, 5:527-533 (1992); Brange, et al., EPO Publication No. 214,826 (published March 18, 1987); and Brange, et al., Current Opinion in Structural Biology, 1: 934-940 (1991). These disclosures are expressly incorporated herein by reference for describing monomeric insulin analogs.

Insulin analogs may also have replacements of the amidated amino acids with acidic forms. For example, Asn may be replaced with Asp or Glu. Likewise, Gln may be replaced with Asp or Glu. In particular, Asn(A18), Asn(A21), or Asp(B3), or any combination of those residues, may be replaced by Asp or Glu. Also, Gln(A15) or Gln(B4), or both, may be replaced by either Asp or Glu.

The term "preservative" refers to a compound added to a pharmaceutical formulation to act as an anti-microbial agent. A parenteral formulation must meet guidelines for preservative effectiveness to be a commercially viable multi-use product. Among preservatives known in the art as being effective and acceptable in parenteral formulations are benzalkonium chloride, benzethonium, chlorohexidine, phenol, m-cresol, benzyl alcohol, methylparaben, chlorobutanol, o-cresol, p-cresol, chlorocresol, phenylmercuric nitrate, thimerosal, benzoic acid, and various mixtures thereof. *See, e.g.,* Wallhäusser, K.-H., *Develop. Biol. Standard,* 24: 9-28 (Basel, S. Krager, 1974). Certain phenolic preservatives, such as phenol and m-cresol, are known to bind to insulin-like molecules and thereby to induce conformational changes that increase either physical or chemical stability, or both [Birnbaum, *et al., Pharmac. Res.* 14:25-36 (1997); Rahuel-Clermont, *et al., Biochemistry* 36:5837-5845 (1997)]. M-cresol and phenol are preferred preservatives in formulations of the monomeric insulin analog proteins used in the present invention.

The term "buffer" or "pharmaceutically acceptable buffer" refers to a compound that is known to be safe for use in insulin formulations and that has the effect of controlling the pH of the formulation at the pH desired for the formulation. Pharmaceutically acceptable buffers for controlling pH at a moderately acid pH to a moderately basic pH include, for example, such compounds as phosphate, acetate, citrate, TRIS, arginine, or histidine.

The term "isotonicity agent" refers to a compound that is tolerated physiologically and imparts a suitable tonicity to a formulation to prevent the net flow of water across the cell membrane. Compounds such as glycerin are commonly used for such purposes at known concentrations. Other acceptable isotonicity agents include salts, *e.g.,* NaCl, dextrose, mannitol, and lactose. Glycerol at a concentration of 12 to 25 mg/mL is preferred as an isotonicity agent.

Administration of Monomeric Insulin Analogs

Monomeric insulin analogs are administered by inhalation in a dose effective manner to increase circulating insulin protein levels and/or to lower circulating glucose levels. Such administration can be effective for treating disorders such as diabetes or hyperglycemia. Achieving effective doses of monomeric insulin analogs requires administration of an inhaled dose of more than about 0.5 µg/kg to about 50 µg/kg monomeric insulin analog protein, preferably about 3 µg/kg to about 20 µg/kg, and most preferably about 7 µg/kg to about 14 µg/kg. A therapeutically effective amount can be determined by a knowledgeable practitioner, who will take into account factors including insulin protein level, blood glucose levels, the physical condition of the patient, the patient's pulmonary status, or the like.

According to the invention, monomeric insulin analogs are delivered by inhalation to achieve rapid absorption of these analogs. Administration by inhalation can result in pharmacokinetics comparable to subcutaneous administration of insulins. Inhalation of monomeric insulin analogs leads to a rapid rise in the level of circulating insulin followed by a rapid fall in blood glucose levels. Different inhalation devices typically provide similar pharmacokinetics when similar particle sizes and similar levels of lung deposition are compared.

According to the invention, monomeric insulin analogs can be delivered by any of a variety of inhalation devices known in the art for administration of a therapeutic agent by inhalation. These devices include metered dose inhalers, nebulizers, dry powder generators, sprayers, and the like. Preferably, monomeric insulin analogs are delivered by a dry powder inhaler or a sprayer. There are a several desirable features of an inhalation device for administering monomeric insulin analogs. For example, delivery by the inhalation device is advantageously reliable, reproducible, and accurate. The inhalation device should deliver small particles, e.g. less than about 10 µm, preferably about 1-5 µm, for good respirability. Some specific examples of commercially available inhalation devices suitable for the practice of this invention are Turbohaler™ (Astra), Rotahaler® (Glaxo), Diskus® (Glaxo), Spiros™ inhaler (Dura), devices marketed by Inhale Therapeutics, AERx™ (Aradigm), the Ultravent® nebulizer (Mallinckrodt), the Acorn II® nebulizer (Marquest Medical Products), the Ventolin® metered dose inhaler (Glaxo), the Spinhaler® powder inhaler (Fisons), or the like.

As those skilled in the art will recognize, the formulation of monomeric insulin analog protein, the quantity of the formulation delivered, and the duration of administration of a single dose depend on the type of inhalation device employed. For some aerosol delivery systems, such as nebulizers, the frequency of administration and length of time for which the system is activated will depend mainly on the concentration of monomeric insulin analog protein in the aerosol. For example, shorter periods of administration can be used at higher concentrations of monomeric insulin analog protein in the nebulizer solution. Devices such as metered dose inhalers can produce higher aerosol concentrations, and can be operated for shorter periods to deliver the desired amount of monomeric insulin analog protein. Devices such as powder inhalers deliver active agent until a given charge of agent is expelled from the device. In this type of inhaler, the amount of monomeric insulin analog protein in a given quantity of the powder determines the dose delivered in a single administration.

The particle size of the monomeric insulin analog protein in the formulation delivered by the inhalation device is critical with respect to the ability of protein to make it into the lungs, and preferably into the lower airways or alveoli.

Preferably, the monomeric insulin analog is formulated so that at least about 10% of the monomeric insulin analog protein delivered is deposited in the lung, preferably about 10% to about 20%, or more. It is known that the maximum efficiency of pulmonary deposition for mouth breathing humans is obtained with particle sizes of about 2 μm to about 3 μm. When particle sizes are above about 5 μm, pulmonary deposition decreases substantially. Particle sizes below about 1 μm cause pulmonary deposition to decrease, and it becomes difficult to deliver particles with sufficient mass to be therapeutically effective. Thus, particles of monomeric insulin analog protein delivered by inhalation have a particle size preferably less than about 10 μm, more preferably in the range of about 1 μm to about 5 μm, and most preferably in the range of about 2 μm to about 3 μm. The formulation of monomeric insulin analog protein is selected to yield the desired particle size in the chosen inhalation device.

Administration of Monomeric Insulin Analogs by a Dry Powder Inhaler

Advantageously for administration as a dry powder, monomeric insulin analog protein is prepared in a particulate form with a particle size of less than about 10 μm, preferably about 1 to about 5 μm, and most preferably about 2 μm to about 3 μm. The preferred particle size is effective for delivery to the alveoli of the patient's lung. Preferably, the dry powder is largely composed of particles produced so that a majority of the particles have a size in the desired range. Advantageously, at least about 50% of the dry powder is made of particles having a diameter less than about 10 μm. Such formulations can be achieved by spray drying, milling, or critical point condensation of a solution containing monomeric insulin analog protein and other desired ingredients. Other methods also suitable for generating particles useful in the current invention are known in the art.

The particles are usually separated from a dry powder formulation in a container and then transported into the lung of a patient via a carrier air stream. Typically, in current dry powder inhalers, the force for breaking up the solid is provided solely by the patient's inhalation. One suitable dry powder inhaler is the Turbohaler™ manufactured by Astra (Södertalje, Sweden). In another type of inhaler, air flow generated by the patient's inhalation activates an impeller motor which deagglomerates the monomeric insulin analog particles. The Dura Spiros™ inhaler is such a device.

Formulations of monomeric insulin analogs for administration from a dry powder inhaler typically include a finely divided dry powder containing monomeric insulin analog protein, but the powder can also include a bulking agent, carrier, excipient, another additive, or the like. Additives can be included in a dry powder formulation of monomeric insulin analog protein, for example, to dilute the powder as required for delivery from the particular powder inhaler, to facilitate processing of the formulation, to provide advantageous powder properties to the formulation, to facilitate dispersion of the powder from the inhalation device, to stabilize the formulation (e.g., antioxidants or buffers), to provide taste to the formulation, or the like. Advantageously, the additive does not adversely affect the patient's airways. The monomeric insulin analog protein can be mixed with an additive at a molecular level or the solid formulation can include particles of the monomeric insulin analog protein mixed with or coated on particles of the additive. Typical additives include mono-, di-, and polysaccharides; sugar alcohols and other polyols, such as, for example, lactose, glucose, raffinose, melezitose, lactitol, maltitol, trehalose, sucrose, mannitol, starch, or combinations thereof; surfactants, such as sorbitols, diphosphatidyl choline, or lecithin; or the like. Typically an additive, such as a bulking agent, is present in an amount effective for a purpose described above, often at about 50% to about 90% by weight of the formulation. Additional agents known in the art for formulation of a protein such as insulin analog protein can also be included in the formulation.

Administration of a dry powder formulation of Humalog®, which is $Lys^{B28}Pro^{B29}$ human insulin, by inhalation is a preferred method for treating diabetes.

Administration of Monomeric Insulin Analogs as a Spray

A spray including monomeric insulin analog protein can be produced by forcing a suspension or solution of monomeric insulin analog protein through a nozzle under pressure. The nozzle size and configuration, the applied pressure, and the liquid feed rate can be chosen to achieve the desired output and particle size. An electrospray can be produced, for example, by an electric field in connection with a capillary or nozzle feed. Advantageously, particles of monomeric insulin analog protein delivered by a sprayer have a particle size less than about 10 μm, preferably in the range of about 1 μm to about 5 μm, and most preferably about 2 μm to about 3 μm.

Formulations of monomeric insulin analog protein suitable for use with a sprayer typically include monomeric insulin analog protein in an aqueous solution at a concentration of about 1 mg to about 20 mg of monomeric insulin analog protein per ml of solution. The formulation can include agents such as an excipient, a buffer, an isotonicity agent, a preservative, a surfactant, and, preferably, zinc. The formulation can also include an excipient or agent for stabilization of the monomeric insulin analog protein, such as a buffer, a reducing agent, a bulk protein, or a carbohydrate. Bulk proteins useful in formulating monomeric insulin analog proteins include albumin, protamine, or the like. Typical carbohydrates useful in formulating monomeric insulin analog proteins include sucrose, mannitol, lactose, trehalose, glucose, or the like. The monomeric insulin analog protein formulation can also include a surfactant, which can reduce or prevent surface-induced aggregation of the monomeric insulin analog protein caused by atomization of the solution in forming an aerosol. Various conventional surfactants can be employed, such as polyoxyethylene fatty acid esters and alcohols, and polyoxyethylene sorbitol fatty acid esters. Amounts will generally range between 0.001 and 4% by weight of the formulation. Especially preferred surfactants for purposes of this invention are polyoxyethylene sorbitan monooleate, polysorbate 80, polysorbate 20, or the like. Additional agents known in the art for formulation of a protein such as insulin analog protein can also be included in the formulation.

Administration of Monomeric Insulin Analogs by a Nebulizer

Monomeric insulin analog protein can be administered by a nebulizer, such as jet nebulizer or an ultrasonic nebulizer. Typically, in a jet nebulizer, a compressed air source is used to create a high-velocity air jet through an orifice. As the gas expands beyond the nozzle, a low-pressure region is created, which draws a solution of monomeric insulin analog protein through a capillary tube connected to a liquid reservoir. The liquid stream from the capillary tube is sheared into unstable filaments and droplets as it exits the tube, creating the aerosol. A range of configurations, flow rates, and baffle types can be employed to achieve the desired performance characteristics from a given jet nebulizer. In an ultrasonic nebulizer, high-frequency electrical energy is used to create vibrational, mechanical energy, typically employing a piezoelectric transducer. This energy is transmitted to the formulation of monomeric insulin analog protein either directly or through a coupling fluid, creating an aerosol including the monomeric insulin analog protein. Advantageously, particles of monomeric insulin analog protein delivered by a nebulizer have a particle size less than about 10 μm, preferably in the range of about 1 μm to about 5 μm, and most preferably about 2 μm to about 3 μm.

Formulations of monomeric insulin analog protein suitable for use with a nebulizer, either jet or ultrasonic, typically include monomeric insulin analog protein in an aqueous solution at a concentration of about 1 mg to about 20 mg of monomeric insulin analog protein per ml of solution. The formulation can include agents such as an excipient, a buffer, an isotonicity agent, a preservative, a surfactant, and, preferably, zinc. The formulation can also include an excipient or agent for stabilization of the monomeric insulin analog protein, such as a buffer, a reducing agent, a bulk protein, or a carbohydrate. Bulk proteins useful in formulating monomeric insulin analog proteins include albumin, protamine, or the like. Typical carbohydrates useful in formulating monomeric insulin analog proteins include sucrose, mannitol, lactose, trehalose, glucose, or the like. The monomeric insulin analog protein formulation can also include a surfactant, which can reduce or prevent surface-induced aggregation of the monomeric insulin analog protein caused by atomization of the solution in forming an aerosol. Various conventional surfactants can be employed, such as polyoxyethylene fatty acid esters and alcohols, and polyoxyethylene sorbital fatty acid esters. Amounts will generally range between 0.001 and 4% by weight of the formulation. Especially preferred surfactants for purposes of this invention are polyoxyethylene sorbitan monooleate, polysorbate 80, polysorbate 20, or the like. Additional agents known in the art for formulation of a protein such as insulin analog protein can also be included in the formulation.

Administration of Monomeric Insulin Analogs by a Metered Dose Inhaler

In a metered dose inhaler (MDI), a propellant, monomeric insulin analog protein, and any excipients or other additives are contained in a canister as a mixture including a liquefied compressed gas. Actuation of the metering valve releases the mixture as an aerosol, preferably containing particles in the size range of less than about 10 μm, preferably about 1 μm to about 5 μm, and most preferably about 2 μm to about 3 μm. The desired aerosol particle size can be obtained by employing a formulation of monomeric insulin analog protein produced by various methods known to those of skill in the art, including jet-milling, spray drying, critical point condensation, or the like. Preferred metered dose inhalers include those manufactured by 3M or Glaxo and employing a hydrofluorocarbon propellant.

Formulations of monomeric insulin analog protein for use with a metered-dose inhaler device will generally include a finely divided powder containing monomeric insulin analog protein as a suspension in a non aqueous medium, for example, suspended in a propellant with the aid of a surfactant. The propellant may be any conventional material employed for this purpose, such as chlorofluorocarbon, a hydrochlorofluorocarbon, a hydrofluorocarbon, or a hydrocarbon, including trichlorofluoromethane, dichlorodifluoromethane, dichlorotetrafluoroethanol and 1,1,1,2-tetrafluoroethane, HFA-134a (hydrofluroalkane-134a), HFA-227 (hydrofluroalkane-227), or the like. Preferably the propellant is a hydrofluorocarbon. The surfactant can be chosen to stabilize the monomeric insulin analog protein as a suspension in the propellant, to protect the active agent against chemical degradation, and the like. Suitable surfactants include sorbitan trioleate, soya lecithin, oleic acid, or the like. In some cases solution aerosols are preferred using solvents such as ethanol. Additional agents known in the art for formulation of a protein such as insulin analog protein can also be included in the formulation.

One of ordinary skill in the art will recognize that the methods of the current invention may be achieved by pulmonary administration of monomeric insulin analogs via devices not described herein.

Pharmaceutical Formulations of Monomeric Insulin Analog Protein

The present invention also relates to a pharmaceutical composition or formulation including monomeric insulin analog protein and suitable for administration by inhalation. According to the invention, monomeric insulin analog protein can be used for manufacturing a formulation or medicament suitable for administration by inhalation. The invention also relates to methods for manufacturing formulations including monomeric insulin analog protein in a form that is suitable for administration by inhalation. For example, a dry powder formulation can be manufactured in several ways, using conventional techniques. Particles in the size range appropriate for maximal deposition in the lower respiratory tract can be made by micronizing, milling, spray drying, or the like. And a liquid formulation can be manufactured by dissolving the monomeric insulin analog protein in a suitable solvent, such as water, at an appropriate pH, including buffers or other excipients.

One particular pharmaceutical composition for a particular monomeric insulin analog protein to be administered through the pulmonary route is Humalog®. Formulations of Humalog® are described by DeFelippis, U.S. Patent No. 5,461,031; Bakaysa, et al. U.S. Patent No. 5.474,978; and Baker, et al. U.S. Patent No. 5,504,188. These disclosures are expressly incorporated herein by reference for describing various monomeric insulin analog formulations. Other formulations include solutions of sterile water alone and aqueous solutions containing low concentrations of surfactants, and/or preservatives, and/or stabilizers, and/or buffers. Additional suitable formulations of monomeric insulin analogs with zinc are known to those of skill in the art.

The present invention may be better understood with reference to the following examples. These examples are intended to be representative of specific embodiments of the invention, and are not intended as limiting the scope of the invention.

## **Examples**

### **Serum Pharmacokinetics of Lys$^{B28}$Pro$^{B29}$ Human Insulin in Beagle Dogs Following Pulmonary Administration of Single Aerosolized Doses**

Aerosols of Lys$^{B28}$Pro$^{B29}$-human insulin (Lys$^{B28}$Pro$^{B29}$-hI), generated from solutions of Lys$^{B28}$Pro$^{B29}$-hI in sterile water, were administered to anesthetized dogs by the pulmonary route through an endotracheal tube via an ultrasonic nebulizer. Serum concentration of immunoreactive Lys$^{B28}$Pro$^{B29}$-hI was determined by validated radioimmunoassay methods.

Six beagle dogs (3 male and 3 female) were used in this study. The animals were housed either two per cage or individually in stainless steel cages with suspended mesh floors. Initially, all dogs were fed approximately 450 g of Purina Certified Canine Diet 5007 each day. Animals were fasted approximately eight hours before dosing. After recovery from anesthesia, food and water were provided *ad libitum* until 48 hours postdose. The initial daily feeding regimen was initiated at 48 hours postdose. At study initiation, the animals weighed between 12.5 and 17.6 kg.

Blood samples were collected at various time points after dosing to determine plasma concentrations of the Lys$^{B28}$Pro$^{B29}$-hI and bioavailability of inhaled material was determined. Dogs were chosen because they are large animals with respiratory tract deposition of particles similar to man.

Pulmonary administration of Lys$^{B28}$Pro$^{B29}$-hI resulted in systemic exposure as indicated by the increased concentrations of immunoreactive Lys$^{B28}$Pro$^{B29}$-hI in the serum of all dogs.

Table 1:

| Serum concentrations of LysB28ProB29-hI (ng/mL) versus time after pulmonary delivery are shown in Table 1: | | | | | | | | | | | | |
|---|---|---|---|---|---|---|---|---|---|---|---|---|
| Time (h$^a$) | 0 | 0.08 | 0.17 | 0.33 | 0.5 | 0.75 | 1 | 1.5 | 2 | 3 | 4 | 6 |
| Dog # (Sex) | | | | | | | | | | | | |
| 26754 (M) | 0.35 | 0.76 | 0.67 | 0.84 | 0.81 | 0.59 | 0.96 | 0.48 | 0.98 | 0.81 | 0.66 | 0.57 |
| 28536 (F) | 0.82 | 3.22 | 3.16 | 2.99 | 1.33 | 2.01 | 1.59 | 0.40 | 2.30 | 0.52 | 0.77 | 0.29 |

[a]abbreviations used: h, hour; M, male; F, female; N, number of animals used in the calculations; SD, standard deviation; SEM, standard error of the mean; BLQ, below the limit of quantitation (<0.25 ng/mL). For the purpose of calculations, BLQ was assigned a value of zero.

Table 1:   (continued)

| Serum concentrations of LysB28ProB29-hI (ng/mL) versus time after pulmonary delivery are shown in Table 1: | | | | | | | | | | | | |
|---|---|---|---|---|---|---|---|---|---|---|---|---|
| 26852 (M) | 0.61 | 2.61 | 2.40 | 3.98 | 2.35 | 2.17 | 2.17 | 1.12 | 0.35 | 0.61 | 2.71 | 0.34 |
| 28911 (F) | 0.83 | 2.61 | 2.14 | 2.27 | 1.67 | 1.90 | 1.79 | 0.59 | 0.53 | 0.28 | 0.30 | BLQ |
| 27258 (M) | N.S.[b] | 1.70 | 2.24 | 2.36 | 1.85 | 1.02 | 0.87 | 0.59 | 0.36 | 0.32 | 0.46 | 0.37 |
| 29245 (F) | 0.60 | 6.01 | 5.34 | 3.81 | 3.21 | 2.32 | 1.44 | 1.25 | 0.68 | 0.27 | 0.35 | 0.33 |
| N | 5 | 6 | 6 | 6 | 6 | 6 | 6 | 6 | 6 | 6 | 6 | 6 |
| Mean | 0.64 | 2.82 | 2.66 | 2.71 | 1.87 | 1.67 | 1.47 | 0.74 | 0.87 | 0.47 | 0.88 | 0.32 |
| SD | 0.20 | 1.78 | 1.54 | 1.16 | 0.83 | 0.70 | 0.50 | 0.36 | 0.74 | 0.22 | 0.92 | 0.18 |
| SEM | 0.09 | 0.73 | 0.63 | 0.47 | 0.34 | 0.28 | 0.20 | 0.15 | 0.30 | 0.09 | 0.37 | 0.07 |

[b]N.S. = No Sample. No serum sample was collected from Dog 27258 prior to dosing (0 h).

Pulmonary administration produced a rapid rise in immunoreactive insulin with peak concentrations ($T_{max}$) occurring in most dogs approximately 5 to 20 minutes after exposure to the aerosol.

Table 2:

| The pharmacokinetic parameters for pulmonarily delivered Lys[B28]Pro[B29]-hI. | | | | | | | | | | |
|---|---|---|---|---|---|---|---|---|---|---|
| Gender | Dog | Weight | Exposed | Total exposed | | $T_{max}$ | $AUC_0^{t'}$ | t' | β | $t_{½}$ |
| | | | Dose | Dose | $C_{max}$ | | | | | |
| | | kg | µg/kg | µg | ng/mL | h | ng*h/mL | h | h(-1) | h |
| M | 28536 | 13.1 | 3.76 | 49.3 | 3.22 | 0.083 | 4.75 | 3.0 | 2.2394 | 0.31 |
| M | 28911 | 13.5 | 7.62 | 102.9 | 2.61 | 0.083 | 2.54 | 1.5 | 0.8607 | 0.81 |
| M | 29245 | 13.9 | 8.71 | 121.1 | 6.01 | 0.083 | 4.89 | 3.0 | 0.9158 | 0.76 |
| F | 26754 | 11.1 | 6.69 | 74.3 | 0.98 | 2 | 4.32 | 6.0 | 0.1977 | 3.51 |
| F | 26852 | 11.9 | 7.08 | 84.3 | 2.36 | 0.33 | 2.17 | 6.0 | 0.8341 | 0.83 |
| F | 27258 | 9.7 | 23.45 | 227 | 3.98 | 0.33 | 8.89 | 2.0 | 1.8245 | 0.38 |
| Mean (M) | | 13.5 | 6.70 | 91.1 | 3.95 | 0.08 | 4.06 | 2.5 | 1.3386 | 0.52 |
| SD | | 0.4 | 2.60 | 37.3 | 1.81 | - | 1.32 | 0.9 | 0.7805 | |
| %CV | | 3.0 | 38.8 | 41.0 | 45.9 | - | 32.5 | 35 | 58.3 | |
| N | | 3 | 3 | 3 | 3 | 3 | 3 | 3 | 3 | 3 |
| Mean (F) | | 10.9 | 12.41 | 129 | 2.44 | 0.89 | 5.13 | 4.7 | 0.9521 | 0.73 |
| SD | | 1.1 | 9.57 | 85.7 | 1.50 | 0.96 | 3.43 | 2.3 | 0.8198 | |
| %CV | | 10.2 | 77.1 | 66.6 | 61.5 | 109 | 67.0 | 49 | 86.1 | |
| N | | 3 | 3 | 3 | 3 | 3 | 3 | 3 | 3 | 3 |
| Mean | (M+F) | 12.2 | 9.6 | 109.9 | 3.19 | 0.48 | 4.59 | 3.6 | 1.1454 | 0.61 |
| SD | | 1.6 | 7.0 | 62.6 | 1.70 | 0.75 | 2.40 | 2.0 | 0.7466 | |
| %CV | | 13.2 | 73.4 | 57.0 | 53.3 | 155 | 52.2 | 55 | 65.2 | |
| N | | 6 | 6 | 6 | 6 | 6 | 6 | 6 | 6 | 6 |
| All Dogs included except | | | | | | | | | | |
| | 27258 | | | | | | | | | |
| Mean | | 12.7 | 6.8 | 86.3 | 3.04 | 0.52 | 3.73 | 3.9 | 1.0095 | 0.69 |
| SD | | 1.2 | 1.8 | 27.4 | 1.85 | 0.84 | 1.29 | 2.0 | 0.7472 | |
| %CV | | 9.2 | 27.3 | 31.7 | 61.1 | 162 | 34.4 | 52 | 74.0 | |

Table 2: (continued)

| The pharmacokinetic parameters for pulmonarily delivered Lys$^{B28}$Pro$^{B29}$-hI. | | | | | | | | | |
|---|---|---|---|---|---|---|---|---|---|
| Gender | Dog | Weight | Exposed | Total exposed | $T_{max}$ | $AUC_0^{t'}$ | t' | β | $t_{1/2}$ |
| All Dogs included except | | | | | | | | | |
| N | | 5 | 5 | 5 | 5 | 5 | 5 | 5 | 5 |

Abbreviations used: kg, kilogram; µg, microgram; ng, nanogram; mL, milliliter; h, hour; $C_{max}$, maximum concentration in serum; $T_{max}$, time to maximum serum concentration; $AUC_0^{t'}$, area under the curve from the time of dosing until a return to baseline; t' "return to baseline"; β, terminal rate constant; $t_{1/2}$, half-life; M, male; F, female; SD, standard deviation; %CV, percent coefficient of variation; N, number of animals used in the calculations.

The data indicated pulmonary administration of aerosolized Lys$^{B28}$Pro$^{B29}$-hI resulted in detectable concentrations of immunoreactive Lys$^{B28}$Pro$^{B29}$-hI in the serum of beagle dogs. Lys$^{B28}$Pro$^{B29}$-hI was absorbed rapidly with mean maximal concentrations achieved in less than 30 minutes. Serum concentrations of immunoreactive Lys$^{B28}$Pro$^{B29}$-hI declined with a mean half-life of around 40 minutes. No appreciable gender differences were noted in the delivery and disposition of Lys$^{B28}$Pro$^{B29}$-hI. Blood glucose values showed a decline to approximately 55% of their control values in fasted dogs following inhalation of Lys$^{B28}$Pro$^{B29}$-hI (Figure 1). The mean lung dose that was required to produce these effects was approximately 7 µg/kg as measured using gamma camera detection of Technetium[99] which was used as a radiolabel in the aerosol droplets. The time taken for the decline in glucose values was slightly less for inhaled Lys$^{B28}$Pro$^{B29}$-hI compared to that observed following subcutaneous injections.

## Claims

1. The use of a monomeric insulin analog protein for the manufacture of a medicament suitable for administration by inhalation.

2. The use according to claim 1, wherein the monomeric insulin analog is selected from the group consisting of modified human insulins wherein:

   a) the amino acyl residue at position B28 is substituted with Lys, Leu, Val, or Ala, and the amino acyl residue at position B29 is Lys or Pro;
   b) the amino acyl residues at positions B28, B29, and B30 are deleted; and
   c) the amino acyl residue at position B27 is deleted.

3. The use of Claim 2, wherein the monomeric insulin analog is LysB28ProB29.

4. The use according to Claim 1, 2, or 3, wherein the medicament is in the form of a solution or an aqueous medium or a suspension or a non-aqueous medium.

5. The use according to Claim 1, 2 or 3, wherein the medicament is in the form of an aerosol.

6. The use according to Claim 1, 2, or 3, wherein the medicament is in the form of a dry powder.

7. The use according to Claim 1, 2, or 3, wherein the monomeric insulin analog has a particle size of less than about 10 microns.

8. The use according to Claim 1, 2, or 3, wherein the monomeric insulin analog has a particle size of about 1 to about 5 microns.

9. The use according to Claim 1, 2, or 3, wherein the monomeric insulin analog has a particle size of about 2 to about 3 microns.

10. The use according to any one of Claims 1 through 9, wherein the monomeric insulin is administered at a dose between about 3 µg/kg to about 20 µg/kg.

11. The use according to any one of Claims 1 through 9, wherein the monomeric insulin is administered at a dose between about 7 μg/kg to about 14 μg/kg.

Figure 1. Glucose response (Mean +/-SE) in Beagle dogs following inhalation of Lys$^{323}$Pro$^{329}$ human insulin.